# EUROPEAN PATENT APPLICATION

(11) **EP 4 559 499 A1**
(43) Date of publication of application: **28.05.2025**
(21) Application number: 23842496.4
(22) Date of filing: 19.07.2023
(51) Int. Cl.: A61M 16/00

(54) **MODULAR VENTILATOR**

(30) Priority: 21.07.2022 ES 202230675
(71) Applicant: Iconic Solutions by Murcia SL, 30100 Murcia (ES)
(72) Inventor: VALVERDE SANCHEZ, Rafael, 30100 MURCIA (ES); ALARCÓN MIÑARRO, Antonio, 30820 ALCANTARILLA (ES); BERNAL GÓMEZ, Francisco, 30005 MURCIA (ES)
(74) Representative: Capitán García, Maria Nuria
(86) International application number: PCT/ES2023/070468
(87) International publication number: WO 2024/018108

(57) **Abstract**

Modular ventilator for assisted mechanical ventilation, comprising a turbine (1), an inspiratory manifold (2), and an expiratory valve (3), comprising a pneumatic module (N) having a first casing inside which it comprises the turbine (1) connected to an equipotential connector of the device, a first electronic control board (4), and the inspiratory manifold (2); an electrical module (E) having a second casing inside which it comprises one or more batteries (8), a single-board computer (SBC) (9) with a control interface and, a second electronic control board (10) connected to the first board (4) and to the control interface; a main structure (19) which can allow for the assembly of both modules, and; a screen (20) connected to the SBC (9), secured to the main structure (19) via securing means that allow it to rotate in two main directions.

## Description

### Technical field of the invention

The present invention corresponds to the technical field of devices for assisted mechanical ventilation, specifically to a ventilator comprising a turbine for generating an air flow, an inspiratory manifold connected to the turbine for conducting said flow therefrom to an inspiratory connection, and an expiratory valve for evacuating the air exhaled by the patient.

### Background of the invention

A mechanical respirator or ventilator is a machine whose function is to replace the spontaneous pulmonary ventilation of a patient with respiratory failure, whether acute or chronic, with mechanical ventilation. The supplied breathing gas is usually enriched with oxygen and can be operated electrically, electromagnetically or pneumatically.

Ventilators are mainly used for sick patients in hospital intensive care units, although they can also be portable, for use in ambulances and emergencies, and domestic, for use in homes.

Mechanical ventilators are considered one of the basic hospital instruments, because while the lack or failure of a patient's natural breathing can cause death, in the same way, the failure of mechanical breathing can also affect the patient, thus considering the ventilator as a vital system.

The importance of this element requires taking the necessary precautions to ensure that mechanical breathing systems are highly reliable and sufficient in number to meet the health demands of the population, thus avoiding endangering the patient.

The problem with these devices arises when a repair is necessary, as this entails a period in which the ventilator is out of service and in most cases this is unaffordable, as there are not enough ventilators available to allow for some reserve units, which means that when it is necessary to repair one of these devices, the situation in hospitals becomes complicated.

In many circumstances, this also leads to increased costs, as it requires investing in a greater number of units to try to avoid these situations, but investing in devices that will be out of use during periods between repairs is very costly.

On the other hand, these ventilators are designed to meet certain mobility conditions, on the one hand, to be able to be used in any location depending on the patient who requires its use, although in practice they are still devices with connected air ducts, with connections to a power supply network, to a hospital air network in most cases... so although they are mobile, they are not to the desirable degree.

On the other hand, they must allow ease of movement in their place of use, to allow the medical team to observe the values indicated on their screen correctly, as well as act on the control of the ventilator by adjusting the values to the needs of the patient. Since these values appear on the control screen, it is necessary, although in practice it is quite uncomfortable, to be continuously rotating the ventilator to achieve visibility of said screen according to the position of the physician in charge.

It is therefore necessary to find a way to solve the problems that arise during the repair periods of these devices, as well as the problems that arise from the need to relocate their position in certain circumstances.

### Description of the invention

The modular ventilator for assisted mechanical ventilation presented here comprises a turbine for generating an air flow, an inspiratory manifold connected to the turbine for conducting said flow therefrom to an inspiratory connection, and an expiratory valve for evacuating the air exhaled by the patient.

This ventilator also comprises a pneumatic module, an electrical module and a main support structure which can allow for the assembly of said modules therein.

The pneumatic module has a first containment casing inside which it comprises at least the turbine connected to an equipotential connector of the device, a first electronic board for controlling its operation, and the inspiratory manifold.

For its part, the electrical module has a second containment casing inside which it comprises one or more batteries, a single-board computer (SBC) comprising an integrated control interface and a second electronic control board with means of connection to the first board and to the control interface.

In addition to the above, this ventilator comprises a screen connected to the SBC for access to the control interface, secured to the main structure via securing means that allow the rotation thereof along two axes corresponding to the two main directions of the screen and which comprises means for compensating the weight of the screen in any tilted position thereof.

The modular ventilator proposed herein represents a significant improvement over the prior art.

This is because a compact and modular design is achieved, which allows for easy manoeuvrability and, above all, in cases where a part of it requires repair or maintenance, it is possible to disconnect it from the rest of the ventilator and exchange it for a similar module, so that the ventilator can continue to operate perfectly while the affected module is repaired, which once repaired is reserved for exchange with the corresponding module of any other ventilator that requires maintenance again.

In this way, it is not necessary to have a large number of reserve ventilators but rather a few modules with which to replace those modules that require maintenance. Thereby achieved is a reduction in costs and, above all, in the inconvenience and discomfort generated in the hospital environment due to the lack of ventilators for patients.

In addition, the screen allows for great versatility of movement thanks to the means securing it to the main structure, so it is not necessary to move the ventilator, thereby avoiding possible and undesirable disconnections of any of its parts.

This is a very important advantage as it provides comfort and ease of work for the medical team, who can quickly achieve visual access to the data on the screen without having to move the entire ventilator, but simply by rotating the screen to the position that is most suitable for the medical team to control the ventilator, without having to move the ventilator itself.

In addition to these advantages, it has other notable aspects such as the fact that it has a turbine as part of the ventilator, so it is able to operate with ambient air and thus does not depend exclusively on the existence of a hospital gas connection. This increases the possibilities and ease of use of the ventilator.

Likewise, its small size and low weight make it a very versatile and easy-to-handle ventilator.

On the other hand, its user interface allows the medical team to set the appropriate ventilation parameters in a simple and effective manner.

It is thus a very effective ventilator that makes it possible to eliminate maintenance time, thanks to the possibility of replacing the electrical or pneumatic module that requires repair with one in perfect condition that allows the ventilator to continue operating normally and also allows great adaptability in the position of the screen, which makes it easy and comfortable to operate the ventilator without having to change its position.

### Brief description of the drawings

To aid a better understanding of the characteristics of the invention according to a preferred example of a practical embodiment thereof, a set of drawings are provided that form an integral part of said description where, for purposes of illustration and in a non-limiting sense, the following is shown:
Figure 1.- Shows a perspective view of the modular ventilator with the screen in the raised position, for a preferred embodiment of the invention.
Figures 2.1, 2.2 and 2.3.- Show front and rear plan and elevation views with the screen of a modular ventilator folded down, for a preferred embodiment of the invention.
Figure 3.- Shows a view of section A-A', wherein the modules of the modular ventilator are represented, for a preferred embodiment of the invention.
Figure 4.- Shows a profile view of the modular ventilator, for a preferred embodiment of the invention.

### Detailed description of a preferred embodiment of the invention

In view of the figures provided, it can be observed how in a preferred embodiment of the invention, the modular ventilator proposed here, for assisted mechanical ventilation, comprises a turbine (1) for generating an air flow, an inspiratory manifold (2) connected to the turbine (1) for conducting said flow therefrom to an inspiratory connection, and an expiratory valve (3) for evacuating the air exhaled by the patient.

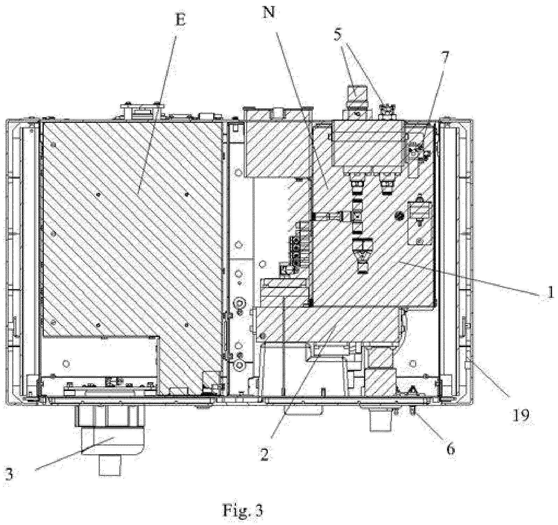

As shown in Figure 3, this modular ventilator further comprises a pneumatic module (N) that has a first containment casing inside which it comprises at least the turbine (1) connected to an equipotential connector of the device, a first electronic board (4) for controlling its operation, and the inspiratory manifold (2).

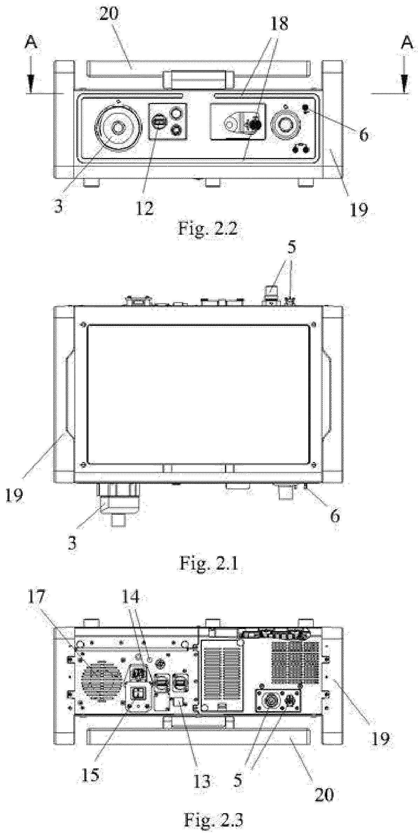

In this preferred embodiment of the invention, the pneumatic module (N) of the ventilator comprises first connections (5) to a source of high and low pressure O₂supply respectively, and a connection thereof to the turbine (1), a connector (6) to an external pneumatic medication nebuliser and a solenoid valve (7) for supplying oxygen to said nebuliser connected to the outlet connector (6) thereto, as can be seen in Figures 2.1 to 2.3.

In addition, in this preferred embodiment, said turbine (1) comprises at least one pressure sensor and one flow rate sensor.

On the other hand, in this preferred embodiment of the invention, the inspiratory manifold (2) comprises an O₂ sensor and one or more emergency and non-return valves, which allow the patient to take air spontaneously if necessary in case of any obstruction.

In this case, in addition, said safety and non-return valves comprise silicone membranes.

Likewise, as shown in Figure 3, the ventilator has an electrical module (E) with a second containment casing inside which it comprises one or more batteries (8), a single-board computer (SBC) (9) comprising an integrated control interface and a second electronic control board (10) with means of connection to the first board (4) and to the control interface.

In this preferred embodiment, the electrical module (E) comprises a third electronic diode board (11) for switching from alternating current to direct current.

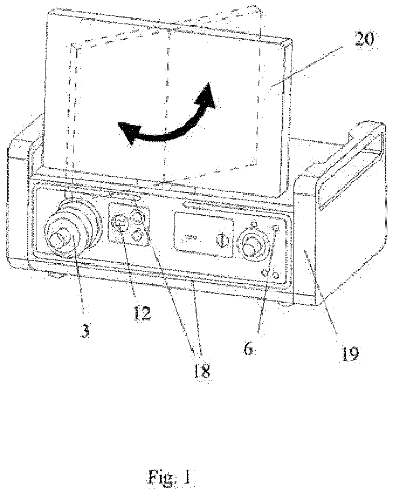

Likewise, as shown in Figure 1, in this preferred embodiment of the invention, said electrical module (E) comprises on the outside of the second casing one or more connection ports, where at least one of them is a USB connection (12), a nurse call port (13), connected to an external alarm system and, second alternating current and direct current connections (14).

In addition, in this case, the electrical module (E) comprises a ventilator on/off switch (15), a speaker (16), a fan (17) and LED devices (18) for displaying ventilator alarms.

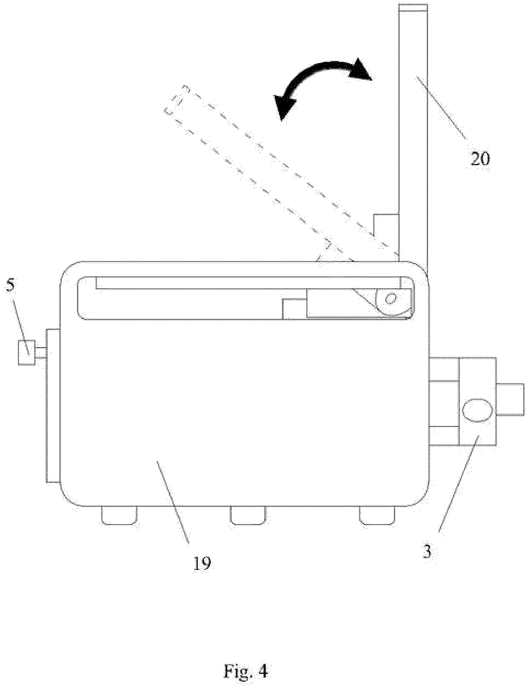

As can be seen in Figures 1 to 4, this ventilator further comprises a main support structure (19) which can allow for the assembly of said modules therein, as well as a screen (20) connected to the USB (12) for access to the control interface, secured to the main structure (19) via securing means that allow rotation thereof along two axes corresponding to the two main directions of the screen (20), as shown in Figures 1 and 4. These securing means comprise means for compensating the weight of the screen (20) in any tilted position thereof.

## Claims

1. Modular ventilator, for assisted mechanical ventilation, comprising a turbine (1) for generating an air flow, an inspiratory manifold (2) connected to the turbine (1) for conducting said flow therefrom to an inspiratory connection, and an expiratory valve (3) for evacuating the air exhaled by the patient, **characterised in that** it comprises
- a pneumatic module (N) having a first containment casing inside which it comprises at least the turbine (1) connected to an equipotential connector of the device, a first electronic board (4) for controlling its operation, and the inspiratory manifold (2);
- an electrical module (E) having a second containment casing inside which it comprises one or more batteries (8), a single-board computer (SBC) (9) comprising an integrated control interface and, a second electronic control board (10) with means for connecting to the first board (4) and to the control interface;
- a main support structure (19) which can allow for the assembly of said modules therein, and;
- a screen (20) connected to the SBC (9) for access to the control interface, secured to the main structure (19) via securing means that allow the rotation thereof along two axes corresponding to the two main directions of the screen (20) and comprising means for compensating the weight of the screen (20) in any tilted position thereof.

2. Ventilator according to claim 1, wherein the pneumatic module (N) comprises first connections (5) to a source of high and low pressure O₂ supply respectively, and a connection thereof to the turbine (1), a connector (6) to an external pneumatic medication nebuliser and a solenoid valve (7) for supplying oxygen to said nebuliser connected to the outlet connector (6) to said nebuliser.

3. Ventilator, according to any of claims 1 or 2, wherein the turbine (1) comprises at least one pressure sensor and a flow rate sensor.

4. Ventilator, according to any of the preceding claims, wherein the inspiratory manifold (2) comprises an O₂ sensor and one or more emergency and non-return valves.

5. Ventilator, according to claim 4, wherein the safety and non-return valves comprise silicone membranes.

6. Ventilator, according to any of the preceding claims, wherein the electrical module (E) comprises a third electronic diode board (11) for switching from alternating current to direct current.

7. Ventilator, according to any of the preceding claims, wherein the electrical module (E) comprises one or more connection ports on the outside of the second casing, wherein at least one of which is a USB connection (12), a nurse call port (13), connected to an external alarm system and second alternating current and direct current connections (14).

8. Ventilator, according to any of the preceding claims, wherein the electrical module (E) comprises a ventilator on/off switch (15), a speaker (16), a fan (17) and LED devices (18) for displaying ventilator alarms.
